# EUROPEAN PATENT SPECIFICATION

(11) **EP 3 302 350 B1**
(45) Date of publication and mention of the grant of the patent: **20.07.2022**
(21) Application number: 16722610.9
(22) Date of filing: 05.05.2016
(51) Int. Cl.: A61K 8/24, A61K 8/25, A61C 19/06, A61Q 11/00, A61K 8/02

(54) **METHOD OF WHITENING TEETH**
VERFAHREN ZUM BLEICHEN VON ZÄHNEN
PROCÉDÉ DE BLANCHIMENT DES DENTS

(30) Priority: 05.06.2015 EP 15170829
(43) Date of publication of application: 11.04.2018
(73) Proprietor: Unilever IP Holdings B.V., 3013 AL Rotterdam (NL); Unilever Global IP Limited, Wirral, Merseyside CH62 4ZD (GB)
(72) Inventor: GROVES, Brian, Joseph, Wirral Merseyside CH63 3JW (GB); LIMER, Adam, John, Wirral Merseyside CH63 3JW (GB); PHILPOTTS, Carole, Jane, Wirral Merseyside CH63 3JW (GB); WILSON, William, John, Wirral Merseyside CH63 3JW (GB)
(74) Representative: Tansley, Sally Elizabeth
(86) International application number: PCT/EP2016/060126
(87) International publication number: WO 2016/192925

(56) References cited:
- WO-A1-2008/068248
- WO-A2-2012/031786
- US-A- 4 083 955
- US-A- 6 159 448
- US-A1- 2006 292 092

## Description

### Field of the Invention

The invention relates to an oral care products for the remineralisation and whitening of teeth.

### Background of the Invention

Teeth are important both functionally and aesthetically. There is a need for strong healthy teeth that appear white and glossy.

Teeth whitening strips are seen as a convenient way of achieving white teeth.

Whitening strips in which the active whitening agent is hydrogen peroxide are disclosed in US5879691 and US6893629. WO 2008/068248 A1 discloses an oral care product comprising a source of calcium ions, a source of phosphate ions, and an insoluble whitening agent for deposition onto the teeth, characterized in that the source of calcium ions and the source of phosphate ions are physically separate prior to the use of the product. The delivery via an adhesive plastic tape is shown, where the compositions are pre-mixed prior to the application.

However, there remains the need for a simple and effective way whiten and strengthen the teeth.

### Summary of the Invention

Accordingly the present invention relates to a method of cosmetically whitening the teeth as defined by the independent claim. Preferred embodiments are described by the dependent claims.

### Detailed Description of the Invention

The delivery device of the invention is preferably flexible, more preferably the device comprises a strip of an orally acceptable flexible material. The surface of the strip is capable of being applied to a tooth surface.

Preferably the device has an elongate shape of a length sufficient that when placed against the front surface of a user's teeth it extends across a plurality of teeth, and of sufficient width that it extends at least from the gumline of the teeth to the crowns of the teeth. The elongate shape is such that it minimises the need for subsequent applications and time to cover all the user's teeth.

In a further embodiment the strip is such that it can be sufficient that when placed against the front surface of a user's teeth it extends across a plurality of teeth, and of sufficient width that it extends at least from the front gumline of the teeth to the crowns of the teeth and to the gumline behind the users teeth leading to total coverage of the teeth above the gumline.

The application device is substantially rectangular in shape.

It is preferable if the device, preferably a strip is formed from an absorbent material, more preferably the device (preferably strip) is a fabric formed from a water soluble, water insoluble, or water hydratable polymer or other material, such as other polymers (e.g., polypropylene, polyethylene, etc.) and cellulose, most preferably the natural fibre is a cotton mix.

If a strip it is preferable if the application strip is greater than 0.001 mm thick and preferably less than 2.5mm thick.

First compositions of the invention comprise water insoluble and/or slightly soluble calcium source. The calcium source is calcium silicate. In a more preferred embodiment, the calcium silicate used is (CaSᵢO₃) whereby the same is made commercially available under the name Microcal ET by Ineos Silicas, Ltd.

In yet another preferred embodiment, the calcium source is insoluble calcium silicate, present as the composite material calcium oxide-silica (CaO-SiO₂) as described in commonly-owned application Publication No. 2008/015117.

The ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition present as the second layer of this invention is typically from 0.1 to 50%, and preferably, from 1 to 30%, and most preferably, from 5 to 20% by weight of the oral care composition based on total weight of the oral care composition and including all ranges subsumed therein.

The first composition is non-aqueous, that is that the composition comprises less than 1 wt% of the total first composition of water, preferably less than 0.5 wt% of water.

The first composition of the invention comprises a whitening source, namely, silicate coated TiO2. Examples of preferred forms of calcium silicate coated titanium dioxide are disclosed in WO2012/031786 and WO2012/031785.

Preferably the level of titanium dioxide is 1-30 wt% or more preferably 5-20 wt% of the second layer.

The second composition comprises a phosphate source, namely, trisodium phosphate and monosodium phosphate.

Typically, the phosphate source makes up from 0.5 to 15%, and preferably, from 2 to 12%, and most preferably, from 4 to 9% by weight of the composition used in the third layer, based on total weight of the composition of the third layer and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

It is preferable if the second composition comprises an aqueous base that is that the composition comprises greater than 50 wt% of the serum composition of water, more preferably greater than 70 wt%.

The oral care compositions described herein may comprise ingredients which are common in the art, such as:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc.;
▪ biomolecules, e.g. bacteriocins, antibodies, enzymes, etc. ;
▪ flavors, e.g., peppermint and spearmint oils;
▪ proteinaceous materials such as collagen;
▪ preservatives;
▪ opacifying agents;
▪ coloring agents like FD&C blue, yellow and/or red dyes/colorants;
▪ pH-adjusting agents;
▪ sweetening agents;
▪ surfactants, such as anionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials
▪ fluoride sources like sodium fluoride, stannous fluoride, sodium; monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
▪ other optional ingredients that may be included are, e.g., bleaching agents such as peroxy compound, e.g., potassium peroxydiphosphate, effervescing systems such as sodium bicarbonate/citric acid systems, color change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer. According to the invention, the second layer comprises glycerin.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

On the final coated device before application to the teeth it is preferable if the thickness of the first backing layer is greater than 0.001mm thick and preferably less than 2.5mm thick, second layer comprising the second composition is less than about 1 mm thick, preferably less than about 0.5 mm thick, and more preferably from about 0.001 to about 0.3 mm thick and third layer (if present) is less than about 1 mm thick, preferably less than about 0.5 mm thick, and more preferably from about 0.001 to about 0.3 mm thick.

The composition used in the layers of the invention are prepared by conventional methods of making oral care formulations. Such methods include mixing the ingredients under moderate shear and atmospheric pressure.

### Mode of Use

The invention provides a method of whitening and remineralising teeth. The method comprises the step of applying a first composition described above to a surface of an application device followed by application of the second composition to same surface. The device is placed on the teeth such that the treated surface of the device is in sustained contact with the teeth. In the context of the present invention sustained contact means the product is left on the teeth for 1 to 30 minutes, preferably, about 5 to 10 minutes before being removed.

Preferably the application of the oral care product of the invention is carried out once daily for a period of several consecutive days, in addition to a regular regime of tooth brushing (preferably at least twice daily).

Typically, use (for a period of about two weeks to one month) of the device with the oral care composition of the present invention will result in a new hydroxyapatite layer on teeth that is from 0.5 to 20 microns, and preferably, from 0.75 to 5 microns, including all ranges subsumed therein.

The invention will now be illustrated by the following non-limiting Examples:

### Examples

A simulated oral fluid was prepared by adding 1.9L of water to a glass beaker. The following materials were added one by one with continuous stirring, allowing sufficient time for each chemical to fully dissolve before adding the next. Sodium chloride 16.07g, sodium hydrogen carbonate 0.7g, potassium chloride 0.448g, potassium hydrogen phosphate 2.56g, magnesium chloride hexahydrate 0.622g, 1M hydrochloric acid 40ml, calcium chloride 0.1998g and sodium sulfate 0.1434g. The pH was adjusted to 7.0 using saturated TRIS buffer and the total volume made up to 2L using a volumetric flask. Human extracted incisors and premolars, obtained for research purposes and obtained with informed consent in accordance with the Human Tissue Act were mounted in plastic cuvettes using the following method. The cuvettes were cut to approximately 15mm height. A commercial fixative (Simplex Rapid) was mixed 2 parts powder to 1 part liquid as described in the manufacturer's instructions. The cuvettes were then completely filled with the resultant solution and left for approximately 10 minutes to allow the fixative to partly set. At this time one tooth root was completely immersed into the fixative, ensuring that the labial side of the tooth was positioned close to the front of the cuvette. Complete setting of the fixative was achieved after 20 minutes. Any exposed dentine is then sealed with clear nail varnish. The teeth are stored in water to prevent dehydration.

A first formulation was prepared by combining the following:

| **Ingredient** | **%w/w** |
|---|---|
| Glycerol | 69.9 |
| Calcium Silicate | 20.0 |
| Calcium silicate coated TiO2 | 10.0 |
| Xanthan | 0.1 |
| Total | 100 |

The glycerol and Xanthan were added to the Esco-Labor 1L mixing vessel and stirring at 65°C for 30 minutes to disperse the xanthan gum. Once the Xanthan has been fully dispersed the remaining powders are added slowly and mixed to remove any lumps.

A second formulation was prepared by combining the following:

**Formulation and processing: Activator solution for the dropper**

| **Ingredient** | **%w/w** |
|---|---|
| Water | 95.1 |
| Trisodium phosphate | 1.63 |
| Monosodium phosphate | 1.37 |
| Cellulose Gum | 1.0 |
| Ethylhexyl glycerine | 0.3 |
| Benzyl Alcohol | 0.3 |
| Phenoxv Ethanol | 0.3 |
| Total | 100 |

Water was added to an Esco-Labor 1L mixing vessel followed by the Sensiva SC50, Ethoxyethanol and Benzyl Alcohol and mixed to disperse. Subsequenrlty SCMC was slowly added to the vessel through the port in the vessel lid and mixed to disperse.

### Example A (not according to the invention)

A product slurry was prepared by separately mixing the components of first and second formulation 50:50 by weight. 7g of mixture was applied to a piece of knitted cotton fabric measuring 10 x 3.5 cm, two samples were prepared. To each sample 5 human teeth were added and the fabric wrapped around the tooth specimens for 30 minutes at 37°C. After the samples were removed from the fabric, rinsed in 40 ml water and mixed gently for 1 minute. Samples were then incubated in simulated oral fluid for 5 hours. The application process was repeated 5 times in total. Colour was measured via chromameter at baseline and after slurry application and incubation. L*a*b* colour parameters were converted to WIO whiteness indices to allow comparison between samples. Colour change is expressed as ΔWIO = WIO(slurry application)-WIO(baseline).

### Example 1

7g of the second formulation was applied to two 10 x 3.5 cm piece of knitted cotton fabric and allowed to impregnate the fabric for 4 hours at room temperature. Immediately before use 3.5g of the first formulation was applied to each fabric sample using a dropping pipette and spread evenly with a spatula. To each sample 5 human teeth were added and the fabric wrapped around the tooth specimens for 30 minutes at 37°C. After the samples were removed from the fabric, rinsed in 40 ml water and mixed gently for 1 minute. Samples were then incubated in simulated oral fluid for 5 hours. The application process was repeated 5 in total. Colour was measured via chromameter at baseline and after slurry application and incubation. L*a*b* colour parameters were converted to WIO whiteness indices to allow comparison between samples. Colour change is expressed as ΔWIO = WIO(slurry application)-WIO(baseline).

| | **1 Application** | **3 Applications** | **5 Applications** |
|---|---|---|---|
| Example A | 4.75 ± 0.69 | 5.53 ± 1.41 | 16.42 ± 3.31 |
| Example 1 | 6.11 ± 1.59 | 9.36 ± 1.43 | 18.66 ± 3.43 |

The examples demonstrate that sequential application of the composition to the device (strip) and applying the strip to the teeth is more effective than forming a pre-mix of the compositions, applying to the strip and applying the device to the teeth.

## Claims

1. A method of cosmetically whitening the teeth **characterised in that** it comprises the following steps:
i) selecting a substantially rectangular application device;
ii) applying composition comprising water, trisodium phosphate, monosodium phosphate and cellulose gum to the surface of the application device
iii) followed by applying a non-aqueous composition comprising calcium silicate, calcium silicate coated TIO₂ and glycerol to the same surface of the application device as the first composition was applied to;
iv) placing a treated surface of device iii) on the teeth for sustained contact.

2. A method according to claim 1 in which the application device is flexible.

3. A method according to any preceding claim in which the application device has an elongate shape of a length that when placed against the front surface of a user's teeth extends across a plurality of teeth, and of width that it extends at least from the gumline of the teeth to the crowns of the teeth.

4. A method according to any previous claim in which the device is formed from an absorbent material.

## Patentansprüche

1. Verfahren zum kosmetischen Aufhellen der Zähne, **dadurch gekennzeichnet, dass** es die folgenden Schritte umfasst:
i) Auswählen einer im Wesentlichen rechteckigen Applikationsvorrichtung;
ii) Auftragen einer Mischung, die Wasser, Trinatriumphosphat, Mononatriumphosphat und Zellulosegummi enthält, auf die Oberfläche der Applikationsvorrichtung,
iii) gefolgt von dem Auftragen einer nichtwässrigen Mischung, die Kalziumsilikat, Kalziumsilikat, das mit TiO₂ beschichtet ist, und Glycerol umfasst, auf dieselbe Oberfläche der Applikationsvorrichtung, auf die die erste Mischung aufgetragen wurde;
iv) Anordnen einer behandelten Oberfläche der Vorrichtung iii) auf den Zähnen für einen andauernden Kontakt.

2. Verfahren nach Anspruch 1, wobei die Applikationsvorrichtung biegsam ist.

3. Verfahren nach einem der vorhergehenden Ansprüche, wobei die Applikationsvorrichtung eine längliche Form mit einer Länge, die sich dann, wenn sie an der Vorderseite der Zähne eines Benutzers angeordnet ist, über mehrere Zähne erstreckt, und mit einer Breite, die sich wenigstens vom Zahnfleischrand der Zähne bis zu den Kauflächen der Zähne erstreckt, hat.

4. Verfahren nach einem der vorhergehenden Ansprüche, wobei die Vorrichtung aus einem absorbierenden Material gebildet ist.

## Revendications

1. Procédé de blanchiment cosmétique des dents **caractérisé en ce qu'**il comprend les étapes suivantes :
i) sélection d'un dispositif d'application substantiellement rectangulaire ;
ii) application d'une composition comprenant de l'eau, phosphate de trisodium, phosphate de monosodium et gomme de cellulose à la surface du dispositif d'application
iii) suivies par l'application d'une composition non aqueuse comprenant du silicate de calcium, TiO2 revêtu de silicate de calcium et glycérol sur la même surface du dispositif d'application sur laquelle la première composition a été appliquée ;
iv) disposition d'une surface traitée du dispositif iii) sur les dents pour un contact prolongé.

2. Procédé selon la revendication 1 dans lequel le dispositif d'application est flexible.

3. Procédé selon l'une quelconque des revendications précédentes dans lequel le dispositif d'application présente une forme allongée d'une longueur telle que lorsque placé contre la surface avant des dents d'un utilisateur il s'étend à travers plusieurs dents, et d'une largeur telle qu'il s'étend au moins à partir de la gencive des dents jusqu'aux couronnes des dents.

4. Procédé selon l'une quelconque des revendications précédentes dans lequel le dispositif est formé à partir d'un matériau absorbant.
